# EUROPEAN PATENT APPLICATION

(11) **EP 2 824 614 A1**
(43) Date of publication of application: **14.01.2015**
(21) Application number: 13176248.6
(22) Date of filing: 12.07.2013
(51) Int. Cl.: G06Q 10/06, G06Q 10/10

(54) **Platform for planning and analyzing sports training for one or more athletes**

(71) Applicant: IQO2 bvba, 9160 Lokeren (BE)
(72) Inventor: Strobbe, Johan Marie Jozef Jaak, 9160 Lokeren (Daknam) (BE); Strobbe, Koenraad Marie Marcel Norbert, 8420 De Haan (BE); Lully, Jorn Hans-Jürgen, 9250 Waasmunster (BE)
(74) Representative: Plas, Axel Ivo Michel

(57) **Abstract**

The invention relates to a platform (1) for planning and analysing sports training for one or more athletes, wherein the platform (1) comprises a tool (2) configured to calculate or to measure for each athlete one or more physiology parameter values, wherein the platform (1) further comprises a training builder module (5) configured to build one or more generic libraries (5b, 6a) comprising a plurality of generic training exercises (5a), a calendar (6b) comprising a training plan for one or more athletes and a training planner module (6) enabling an authorized user of the platform (1) to add a generic training exercise (5a) from the one or more generic libraries (5b, 6a) to the calendar (6b) of one or more athletes, the training planner module (6) further being adapted to automatically individualize the generic training exercise (5d) put into the calendar (6b) of the one or more athletes using the one or more physiology parameter values (2) respectively calculated or measured for the one or more athletes, resulting in a calendar (6b) with an individualized training plan (6b) for the respective athlete.

## Description

### Field of the Invention

The present invention generally relates to a platform for planning and analysing sports training for one or more athletes. These athletes can be trained as well as untrained and can be professional as well as non-professional.

This invention more specifically relates to such a platform comprising a tool configured to calculate or to measure for each athlete one or more physiology parameter values.

### Background of the Invention

At present, tools have been developed that are coupled to portable devices that are capable of monitoring the performance of an individual supporting such a portable device during a work-out. These portable devices may transmit and receive a variety of information to and from remote server computers via networks in order to assist the individual in their exercise goals. Sports brands like Nike and Adidas developed such tools.

In US 2012/0015779 for instance, Adidas AG describes a computer-implemented method for providing a new workout for an athlete utilizing a fitness monitoring service. The method includes the steps of granting an athlete access to an account of the fitness monitoring service, maintaining a schedule of work-outs for the athlete to complete in association with the account, receiving the new workout from a coach, adding the new workout to the schedule of workouts and exchanging information relation to the new work-out with a portable fitness monitoring device.

In US 2006/0136173 for example, Nike Inc. describes athletic performance monitoring systems and methods, many of which utilize, in some manner, global positioning satellite ("GPS") data, provide data and information to athletes and/or to equipment used by athletes during an athletic event. Such systems and methods may provide route information to athletes and/or their trainers, e.g., for pre-event planning, goal setting, and calibration purposes. Such systems and methods optionally may provide real time information to the athlete while the event takes place, e.g., to assist in reaching the pre-set goals. Additionally, data and information collected by such systems and methods may assist in post-event analysis for athletes and their trainers, e.g., to evaluate past performances and to assist in improving future performances.

The disadvantage of these tools is that planning training for multiple team members using such existing tools remains a time-consuming and repetitive job for coaches and technical staff.

Accordingly, there is a need to provide a platform for planning and analysing sports training that enables efficient coaching, i.e. planning and analysing of training, of an athlete or a team of athletes by a multidisciplinary team of amongst others physiologists, technical staff, coaches, paramedics, mental coaches, etc. There is a further need to provide such a platform that enables planning of training for multiple team members in a time-efficient way.

### Summary of the Invention

In the present invention, the above identified shortcomings of existing tools are resolved by providing a platform for planning and analysing training for one or more athletes, the platform comprising a tool configured to calculate or to measure for each athlete on or more physiology parameter values, wherein the platform further comprises a training builder module configured to build one or more generic libraries comprising a plurality of generic training exercises, a calendar comprising one or more training plan for one or more athletes, a training planner module enabling an authorized user of the platform to add a generic training exercise from the one or more generic libraries to the calendar of one or more athletes, the training planner module further being adapted to automatically individualize the generic training exercise put into the calendar of the one or more athletes using the one or more physiology parameter values respectively calculated or measured for the one or more athletes, resulting in a calendar with an individualized training plan for the respective athlete(s).

The advantage of this platform is that a multidisciplinary platform is obtained which is usable by physical therapists, technical staff, coaches, paramedics, etc. surrounding an athlete or a team of athletes. It furthermore enables a team coach to efficiently plan training exercises for multiple team members from generic training exercises that are automatically individualized for respective team members. This avoids repetition of making up training schemes providing is serious time savings for a team coach.

In a preferred embodiment of a platform according to the invention, the one or more physiology parameter values comprise an anaerobic threshold of the one or more athletes, wherein the platform comprises a training zones module which is configured to define a plurality of training zones of the one or more athletes differing in intensity of the training, each of the training zones being calculated as a percentage of the anaerobic threshold.

The anaerobic threshold (ANT) is defined as the training intensity at which there is a rapid rise in blood lactate, indicating the upper limit of equilibrium between lactate production and clearance. This ANT consequently forms the transition between the aerobe and the anaerobe lactic energy supply.

The ANT varies from person to person, and, within a given individual, sport to sport. Untrained individuals have a low ANT (approximately 55 % of V02 max), and elite endurance athletes, a high ANT (approx. 80 - 90% of V02 max). V02 max is the maximal amount of oxygen that can be consumed by an athlete during one minute on sea level. The oxygen consummation is linearly related to the energy consumption. This means that when the oxygen consumption is measured, also indirectly the maximal capacity of an athlete in order to deliver aerobic efforts is measured. 95 % of the efforts of an endurance athlete consist out of aerobic effort. The higher the V02 max, the more talent an athlete has to be an endurance athlete. The later an athlete reaches the ANT, the better its endurance. This point is also called the maximum lactate steady-state. An athlete can train its body to remove lactate better and to juice up the aerobic mitochondrial enzymes, thus raising the ANT.

Efforts below the ANT can be sustained for quite some time. Efforts with an intensity above the ANT will have to be quickly suspended. Accumulation of lactate limits performance to periods for 30 seconds to 15 minutes. Exhaustion results through the disturbance of the internal biochemical environment of the working muscle and whole body caused by a high acidosis.

In a more preferred embodiment of a platform according to the invention, the one or more training zones are sports specific, wherein the percentage of the anaerobic threshold related to a respective training zone is determined on the basis of one or more predetermined definitions that are dependent from the respective sport.

In a possible embodiment of a platform according to the invention, the platform comprises a training intensity calculator module that is configured to calculate the anaerobic threshold of a respective athlete on the basis of predefined fitness levels that are related to the age, the gender, the resting heart rate and the maximum heart rate of the respective athlete.

In another possible embodiment of a platform according to the invention, the platform comprises a lactate test module that is configured to analyse the relation between the power, the speed, the heart rate and the lactate value of the tested athlete, out of which the anaerobic threshold of the respective athlete is determinable.

In still another possible embodiment of a platform according to the invention, the platform comprises a functional threshold module that is configured to analyse data of the tested athlete monitored by a monitoring device during a training exercise at an optimal intensity, out of which the anaerobic threshold of the respective athlete is determinable.

This monitoring device preferably comprises
- a power monitor adapted to measure the power of the legs of the respective athlete performed during the training and the heart rate of the respective athlete during the training in case the sports discipline is cycling; and
- a speed monitor adapted to measure the speed and the heart rate of the respective athlete during the training in case the sports discipline is running or swimming.

In a favourable embodiment of a platform according to the invention, the training planner module is arranged to calculate a mesocycle from a respective training plan by summing volume and/or intensity of one or more training exercises in the individualized training plan.

A mesocycle represents a phase of training with a duration of between 2 - 6 weeks or micro-cyles, but this can depend on the sporting discipline. During the preparatory phase, a mesocycle commonly consist of 4 - 6 micro-cycles, while during the competitive phase it will usually consist out of 2 - 4 micro-cycles depending on the competition's calendar. The goal of the planner is to fit the mesocycles into the overall plan timeline-wise to make each mesocycle end on one of the phases and then to determine the workload and type of work of each cycle based on where in the overall plan the given mesocycle falls. The goal in mind is to make sure the body peaks for the high priority competitions by improving each cycle along the way.

In an advantageous embodiment of a platform according to the invention, the training planner module is arranged to determine a macrocycle from the individualized training plan representing a year planning of volumes, intensity, period and/or type of training exercise.

A macrocycle refers to an annual plan that works towards peaking for the goal competition of the year.

A further disadvantage of the known monitoring devices that are used to collect data relating to athletic performance, for instance monitoring devices of RIM, Polar, SRM, Garmin, CycleOps, Suunto, etc, is that each of these devices has their own format and way of recording data. The SRM training system devices for instance register per second, Garmin devices apply smart recording, etc. As a consequence, data from multiple team members using different devices are difficult to compare.

This disadvantage is overcome in an advantageous embodiment of a platform according to the invention, comprising a device data module arranged for extracting data from one or more monitoring devices carried by an athlete during one or more training exercises, wherein the device data module is furthermore arranged to convert the data extracted from the one or more monitoring devices to a proprietary format usable in the different modules of the platform.

This provides in a platform that is independent from the device(s) used by the athlete(s) and leads to a uniform analysis of all devices through a proprietary format.

In a preferred embodiment of a platform according to the invention, the platform comprises an analytic module configured to compare data extracted from the one or more monitoring devices with data out of the data with the individualized training plan of the respective athlete.

This gives the opportunity to optimize the training plan of an athlete based on these comparisons.

The data extracted from the one or more monitoring devices preferably comprise the volume and/or the intensity of the training exercise as performed by the respective athlete.

Furthermore, at present, several web-based applications are already available for planning and analysing sports training. Examples thereof are TrainingPeaks and Asicoach.com. Also several device manufacturers offer such web-based applications. Examples thereof are polarpersonaltrainer.com of Polar and Garmin Connect of Garmin.

These web-based applications however require network connectivity. The athlete must be regularly online which is not possible when the athlete is following a training plan at a remote location where there is no or poor network connectivity.

It is a further need to provide such a platform that is independent from network connectivity and thus can be used both online and offline.

This need is fulfilled by providing a platform according to the invention, comprising an application that is run on a desktop pc, wherein the application is configured to be run at the moment a user thereof is offline, as well as it can be run at the moment the user is online.

Preferably, the application is configured to upload data from one or more of the monitoring devices, wherein the application is configured to automatically upload the data uploaded from the one or more monitoring devices to a server or the web when the user of the application is online or as soon as a user of the application has signed in the application to be online.

The application furthermore is preferably configured to allow multiple users to use the application at the same time online as well as offline.

### Brief Description of the Drawings

Fig. 1 illustrates a flow chart of the different modules of an embodiment of a platform according to the invention.

Fig. 2 illustrates a flowchart diagram of the common fields in all database datasets to support sync online / offline.

### Detailed Description of Embodiment(s)

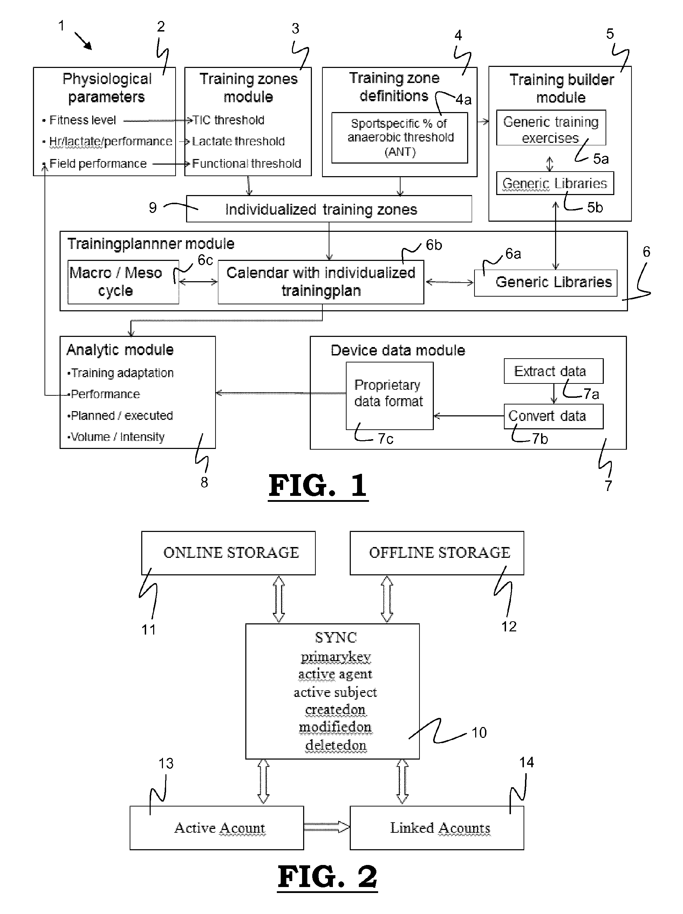

As can be seen in figure 1, a platform (1) according to the invention for planning and analysing sports training comprises a tool (2) configured to calculate or to measure for each athlete one or more physiology parameter values. These one or more physiology parameter values preferably comprise the anaerobic threshold (ANT) (as explained in more detail above).

This ANT is determinable in three different ways, i.e.
1. by means of a training intensity calculator (TIC) module that is configured to calculate the ANT of a respective athlete on the basis of predefined fitness levels (poor, good, moderate, excellent) that are related to the age, the gender, the resting heart rate and the maximum heart rate of the respective athlete;
2. using a lactate test module that is configured to analyse the relation between the power, the speed, the heart rate and the lactate value of the tested athlete, out of which the ANT is determinable;
3. by means of a functional threshold module that is configured to analyse data of the tested athlete monitored by a monitoring device (not shown on the figures) during a training at an optimal intensity (field performance), out of which the ANT of the respective athlete is determinable.

In case the sports discipline is cycling, this monitoring device preferably comprises a power monitor adapted to measure the power of the legs of the respective athlete performed during the training and the heart rate of the respective athlete during the training. In case the sports discipline is running or swimming, the monitoring device comprises a speed monitor adapted to measure the speed and the heart rate of the respective athlete during the training.

The platform (1) according to the invention comprises a device data module (7) to extract data (7a) from these monitoring devices carried by an athlete during one or more training exercises. This device data module (7) is furthermore arranged to convert the data (7b) extracted from these one or more monitoring devices to a proprietary data format (7c) that can be used in the different modules (2 - 8) of the platform (1) and that is independent from the different monitoring devices.

The data of the monitoring device(s) is stored in a uniform format for all devices and sports. In table 1, the fields of a workout dataset as stored in the uniform dataset is shown.

**Table 1**

| **COLUMN** | **UNIT** | **PRECISION** | |
|---|---|---|---|
| ROWNUMBER | N/A | | |
| ELAPSEDSECONDS | seconds | 3 | Elapsed Time |
| HEARTRATE | Bpm | 0 | Heart rate |
| POWER | Watt | 0 | Power |
| SPEED | Km/h | 3 | Speed |
| DISTANCE | Km | 3 | Distance |
| CADENCE | Rpm | 0 | Cadence |
| TORQUE | Nm | 0 | Torque (Power = Torque * Angular Velocity) |
| ALTITUDE | Meter | 1 | Altitude |
| SLOPE | % | 2 | Gradient |
| AIRPRESSURE | Pa | 0 | Air Pressure |
| TEMPERATURE | Celcius | 1 | Temperature |
| POWERINDEX | N/A | 0 | Power Distribution |
| LATITUDE | Degrees | 7 | GPS Position Latitude |
| LONGITUDE | Degrees | 7 | GPS Position Longitude |
| LAP | N/A | 0 | Splits/Intervals Markers |
| ID | N/A | 0 | Internal Use |

This format is extendable and adaptable. If the format changes or field are added, the format stored on a server or locally is automatically converted to the latest format. When data are stored, the data are first encoded to minimize the number of bytes needed to store each row in the dataset. This is done by scanning the data, calculating the minimum and maximum values of each column. The ID, precision, minimum and maximum values are stored in the header of the encoded data. The number of bytes required to store the data for a column is calculated from (maximum - minimum)*10^precision and the actual data is offset from the minimum value. This ensures that a minimal amount of bytes is used for each row without sacrificing the compression ratio in the next step. Only columns with data are stored. The format is a variable format since the metadata (ID and precision for example) of the actual columns is stored. Finally, an LZMA (Lempel-Ziv-Markov chain algorithm) compression algorithm is applied to the encoded data before storing it in the database. The data is also versioned, and changing compression algorithms are possible.

The platform (1) according to the invention comprises a training zones module (3) which is configured to enable a user of the platform (1) to select the way in which the ANT is determined.

The platform (1) furthermore comprises a training zone definitions module (4) which is configured to define a plurality of training zones of the one or more athletes differing in intensity of the training. Each of these training zones is fixedly defined as a percentage of the ANT and is sports specific. The platform (1) comprises a training zone definitions module (4) comprising these predefined fixed training zone definitions. After the determined ANT has been determined by one of the methods as described above, this ANT is then used by the training zone definitions module (4), or in other words the training zone definitions are applied in the training zones module (3) resulting in individualized training zones (9).

In table 2 below, an example of a number of training zones for the sports discipline running is shown, in which for each of these training zones the range of the heart rate (expressed in beats per minute), the percentage of the ANT heart rate, the percentage of the maximum heart rate, the speed (in km/h) and the percentage ANT speed is shown.

**Table 2**

| Training zone | Heart rate (beats per minute) | % Anaerobic threshold hear rate | % maximum heart rate | Speed (km / h) | % Anaerobic threshold speed |
|---|---|---|---|---|---|
| Active Recovery | < 134 | < 75 | < 72 | 0,0 - 9,2 | 0-70 |
| Extensive endurance - low | 134 - 150 | 75-84 | 72 - 80 | 9,2 - 10,6 | 70, -80 |
| Extensive endurance - high | 150 - 161 | 84-90 | 80 - 86 | 10,6-11,9 | 80-90 |
| Intensive endurance | 161 - 175 | 90 - 98 | 86 - 94 | 11,9- 12,5 | 90 - 95 |
| Threshold | 175 - 184 | 98 - 103 | 94-99 | 12,5 - 13,9 | 95 - 105 |
| V02 max | > 184 | > 103 | > 99 | 13,9 - 15,2 | 105-115 |
| Anaerobic capacity | Not available | Not available | Not available | 15,2 - 19,8 | 115-150 |
| Neuromuscular power | Not available | Not available | Not available | 19,8 - 79,2 | 150 - 600 |

The platform (1) according to the invention comprises a training builder module (5) configured to build one or more generic libraries (5b, 6a) comprising a plurality of generic training exercises (5a).

The platform (1) also comprises a training planner module (6) that enables an authorized user of the platform (1) to make up a training plan for an athlete or a team of athletes in the calendar of the respective athlete(s) starting from generic training exercises (5a) that are stored in one or more generic libraries (5a, 6b). A training plan can comprise one or a plurality of workouts / training exercises. These generic training exercises (5a) constitute blocks that can be added to the calendar (6b) and are categorized, such as warm-up, cool-down, exercise, exercise group, repeat group, etc. enabling semi-automatic recognition, addition and classification of training exercises. Adding a generic training exercise (5a) to the calendar (6b) of an athlete preferably is done by means of a drag-and-drop user interface, this method however not excluding any other suitable method to add generic training exercises (5a) to the calendar. When a generic training exercise (5a) is added to the athlete's calendar, this generic training exercise (5a) is automatically adapted in view of the ANT of the athlete that is upfront calculated or measured by means of one of the ways as disclosed above and determined by the user of the platform (1) in the training zones module (3) as already described above. In this way, a calendar with an individualized training plan (6b) is obtained.

The training planner module (6) furthermore is arranged to calculate a mesocycle (6c) from the respective calendar with the individualized training plan (6b) by summing volume and/or intensity of one or more training exercises in the individualized training plan. Also a macrocycle can be determined from the individualized training plan representing a year planning of volumes, intensity, period and/or type of training exercises. The definitions of mesocycle and macrocycle are already given above.

The platform (1) according to the invention also comprises an analytic module (8) configured to compare data extracted from the one or more monitoring devices, preferably the volume and/or the intensity of the training exercise(s) as performed by the respective athlete, with the data out of the planned individualized training plan.

This analytic module (8) is furthermore adapted to calculate overtraining of an athlete. Overtraining is a physical, behavioral, and emotional condition that occurs when the volume and intensity of an athlete's training exercise exceeds their recovery capacity. They cease making progress, and can even begin to lose strength and fitness. The platform (1) according to the invention can comprise a display module (not shown on the figures) adapted to visualize fatigability of an athlete.

The platform (1) according to the invention preferably comprises an application that is run on a desktop pc, wherein the application is configured to be run at the moment a user thereof is offline, as well as it can be run at the moment the user is online. The application is further configured to allow multiple users to work at the same moment offline in the same file of a respective athlete.

This application preferably is configured to upload data from one or more of the monitoring devices, wherein the application is configured to automatically upload the data uploaded from the one or more monitoring devices to a server or the web when the user of the application is online or as soon as a user of the application has signed in the application to be online.

This application furthermore preferably is configured to allow multiple users to use the application at the same time online as well as offline.

As can be seen in figure 2, to enable sync ability, all datasets in the database share common items. All ID's are GUIDs (global unique identifiers) through which it doesn't matter if they are made local or remote.

The common fields in all database datasets to support sync (10) online/offline are:
- primary key id: a GUID which identifies the record;
- activeagent: a GUID which identifies the active user (user account used to log in);
- activesubject: a GUID which identifies the active athlete;
- createdon: a timestamp when a record is created;
- modifiedon: a timestamp when a record is modified;
- deletedon: a timestamp when a record is deleted.

Accessing Online or Offline data is transparent to the software by the use of interfaces. The interface describes the functions of the storage object, there can be multiple implementations of this interface, e.g. online storage (11) and offline storage (12). The storage technology could be changed altogether by writing a new implementation.

There are two entities which define to which user account data is linked, i.e.
- activeagent is the logged in user account;
- activesubject is the user account used for training data (workouts, training zones, calendar, periodic, etc.).
After signing in, activeagent is equal to activesubject until the user selects another athlete account from the client management. A coach can for example have multiple linked accounts (14) of several athletes.

In a single user environment, if the user does not have linked accounts (14), activeagent will always be equal to active subject. In a multi-user environment, where linked accounts (14) are linked to one or more other active accounts (13), they will refer to different user accounts, or in other words, one user changes data in the account of another user. On the moment a user logs in, an active account (13) is obtained.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A platform (1) for planning and analysing sports training for one or more athletes, wherein the platform (1) comprises a tool (2) configured to calculate or to measure for each athlete one or more physiology parameter values, **CHARACTERIZED IN THAT** the platform (1) further comprises
- a training builder module (5) configured to build one or more generic libraries (5b, 6a) comprising a plurality of generic training exercises (5a);
- a calendar comprising a training plan for one or more athletes;
- a training planner module (6) enabling an authorized user of the platform (1) to add a generic training exercise (5a) from the one or more generic libraries (5b, 6a) to the calendar of one or more athletes, the training planner module (6) further being adapted to automatically individualize the generic training exercise (5d) put into the calendar of the one or more athletes using the one or more physiology parameter values (2) respectively calculated or measured for the one or more athletes, resulting in a calendar with an individualized training plan (6b) for the respective athlete(s).

2. A platform (1) according to claim 1, **CHARACTERIZED IN THAT** the one or more physiology parameter values (2) comprise an anaerobic threshold of the one or more athletes, and **in that** the platform (1) comprises a training zones definition module (4) which is configured to define a plurality of training zones of the one or more athletes differing in intensity of the training, each of the training zones being calculated as a percentage of the anaerobic threshold.

3. A platform (1) according to claim 2, **CHARACTERIZED IN THAT** the one or more training zones are sports specific, and **in that** the percentage of the anaerobic threshold related to a respective training zone is determined on the basis of one or more predetermined definitions that are dependent from the respective sport.

4. A platform (1) according to claim 2 or 3, **CHARACTERIZED IN THAT** the platform (1) comprises a training intensity calculator module that is configured to calculate the anaerobic threshold of a respective athlete on the basis of predefined fitness levels that are related to the age, the gender, the resting heart rate and the maximum heart rate of the respective athlete.

5. A platform (1) according to claim 2 or 3, **CHARACTERIZED IN THAT** the platform (1) comprises a lactate test module that is configured to analyse the relation between the power, the speed, the heart rate and the lactate value of the tested athlete, out of which the anaerobic threshold of the respective athlete is determinable.

6. A platform (1) according to claim 2 or 3, **CHARACTERIZED IN THAT** the platform (1) comprises a functional threshold module that is configured to analyse data of the tested athlete monitored by a monitoring device during a training exercise at an optimal intensity, out of which the anaerobic threshold of the respective athlete is determinable.

7. A platform (1) according to claim 6, **CHARACTERIZED IN THAT** the monitoring device
- in case the sports discipline is cycling, comprises a power monitor adapted to measure the power of the legs of the respective athlete performed during the training and the heart rate of the respective athlete during the training;
- in case the sports discipline is running or swimming, comprises a speed monitor adapted to measure the speed and the heart rate of the respective athlete during the training.

8. A platform (1) according to any one of claims 1 to 7, **CHARACTERIZED IN THAT** the training planner module (6) is arranged to calculate a mesocycle (6c) from a respective training plan by summing volume and/or intensity of one or more training exercises in the training plan.

9. A platform (1) according to claim 8, **CHARACTERIZED IN THAT** the training planner module (6) is arranged to determine a macrocyle (6c) from one or more individualized training plan representing a year planning of volumes, intensity, period and/or type of training exercise.

10. A platform (1) according to any one of claims 1 to 9, **CHARACTERIZED IN THAT** the platform (1) comprises a device data module (7) arranged for extracting data (7a) from one or more monitoring devices carried by an athlete during one or more training exercises, wherein the device data module (7) is furthermore arranged to convert the data (7b) extracted from the one or more monitoring devices to a proprietary format (7c) usable in the different modules (2 - 8) of the platform (1).

11. A platform (1) according to claim 10, **CHARACTERIZED IN THAT** the platform (1) comprises an analytic module (8) configured to compare data extracted from the one or more monitoring devices with data out of the calendar with the individualized training plan (6b) of the respective athlete.

12. A platform (1) according to claim 10 or 11, **CHARACTERIZED IN THAT** the data extracted from the one or more monitoring devices comprise the volume and/or the intensity of the training exercise as performed by the respective athlete.

13. A platform (1) according to any one of claims 1 to 12, **CHARACTERIZED IN THAT** the platform (1) comprises an application that is run on a desktop pc, wherein the application is configured to be run at the moment a user thereof is offline, as well as it can be run at the moment the user is online.

14. A platform (1) according to claim 13, **CHARACTERIZED IN THAT** the application is configured to upload data from one or more of the monitoring devices, wherein the application is configured to automatically upload the data uploaded from the one or more monitoring devices to a server or the web when the user of the application is online or as soon as a user of the application has signed in the application to be online.

15. A platform (1) according to claim 13 or 14, **CHARACTERIZED IN THAT** the application is configured to allow multiple users to use the application at the same time online as well as offline.
